Europäisches Patentamt

European Patent Office

Office européen des brevets

(11) Publication number: **0 278 660 B1**

(19)

# EUROPEAN PATENT SPECIFICATION

(12)

(45) Date of publication of patent specification: **07.10.92**

(51) Int. Cl.⁵: **A61K 7/00**, A61K 9/10, B01F 17/00

(21) Application number: **88300856.7**

(22) Date of filing: **02.02.88**

(54) **Microemulsions.**

(30) Priority: **03.02.87 IE 283/87**

(43) Date of publication of application:
**17.08.88 Bulletin  88/33**

(45) Publication of the grant of the patent:
**07.10.92 Bulletin  92/41**

(84) Designated Contracting States:
**AT CH DE ES FR GB GR IT LI LU NL SE**

(56) References cited:
**FR-A- 2 470 595
FR-A- 2 553 661**

**CHEMICAL ABSTRACTS, vol. 102, 1985, page
391, abstract no. 84795y, Columbus, Ohio,
US; R.L. VENABLE: "Microemulsions with ex-
cellent water solubilizing capacity at high
hydrocarbon levels with quaternary ammo-
nium salts as surfactants", & JAOCS. J. AM.
OIL CHEM. SOC. 1985, 62(1), 128-33**

(73) Proprietor: **STIEFEL LABORATORIES
(IRELAND) LIMITED
Finisklin Industrial Estate
Sligo(IE)**

(72) Inventor: **Bourke, Geoffrey Michael Francis
50 Thornhill
Sligo(IE)**
Inventor: **O'Rourke, Brian
2 Carton Court
Sligo(IE)**
Inventor: **Singleton, James Gerard
322 Cartron Point
Sligo(IE)**

(74) Representative: **Sheard, Andrew Gregory et al
Kilburn & Strode 30, John Street
London WC1N 2DD(GB)**

**Description**

This invention relates to clear, oily, homogenous microemulsions suitable for use in topically applied health care and personal care products. In particular, the invention relates to the use of such microemulsions fragrances, cosmetics, hair care products, toiletries and pharmaceuticals.

Microemulsions are emulsions which form spontaneously when their component phases are brought into contact. The very small dispersed droplet size (typically $10^{-8}$ to $10^{-7}$ m) confers on the emulsion an inordinate stability and gives it special consideration in the fields of dispersion, detergency, drug delivery, etc., as they are invariably translucent, and frequently transparent, microemulsions have an attractive appearance which enhances their popularity. Whereas macroemulsions are strongly thermodynamically unstable, microemulsions may form irreversibly. Microemulsions, which are sometimes referred to as "swollen micelles" represent the transition between macroemulsion proper and the solubilised state which results from detergent action.

The major restriction on the expanded use of microemulsions in dispersion technology is that they are frequently very difficult if not impossible to produce and a wide range of surfactants must be screened in their potential creation. Such investigation is clearly time-consuming and expensive. More often than not serendipity is the author of the stable formulation.

Many Patents covering the preparation of microemulsions stabilised by nonionic surfactants and by mixtures of anionic soaps and fatty alcohol cosolubiliser/cosurfactant have been issued. Few Patents have been issued governing cationic-stabilised microemulsions, still less in water-in-oil systems. Whereas anionic and nonionic surfactants find widespread use in detergency, wetting, dispersion, etc., cationic surfactants in general have poor detergent properties, generate hydrophobic surfaces through strong interfacial adsorption and feature in pharmaceutical and sanitational technologies largely on account of their antibacterial properties.

Venable, R. L. (1985) in J.A.O.C.S., Vol. 62, No. 1 describes water-in-oil microemulsions comprising a mixture of heptane or toluene and pentanol, water and various quaternary ammonium salt cationic surfactants. The paper describes the effect of the hydrocarbon chain length of the quaternary ammonium salts on the water solubilizing capacity of the microemulsions. No use is indicated for the microemulsions studied.

FR-A-2 553 661 describes oil-in-water microemulsions for parenteral administration stabilised by anionic surfactants of the type which are comprised of a salt of an aliphatic organic acid containing 10-22 carbon atoms with an inorganic or organic base. FR-A-2 470 595 describes cosmetic compositions for the care and treatment of the skin comprising three-component, oil-in-water emulsions which form opaque, white or semi-white creams or lotions.

It is an object of the present invention to provide clear, stable microemulsions for topical application which can easily and inexpensively be produced and which have a wide range of applications as herein described.

According to the invention there is provided a clear, homogenous microemulsion for topical application to the hair and skin comprising a hydrophobic oily phase of at least 20% by weight, 0.01-20% by weight of a hydrophilic phase and 0.01-20% by weight of a cationic quaternary ammonium surfactant, from 0.1 to 10% by weight of a dispersant of high HLB of at least 9 and sufficient cosurfactant to generate and secure homogeneity and clarity of the microemulsion.

We have discovered that clear, frequently colourless microemulsions suitable for topical application in health care and personal care products can be prepared through the incorporation of low to medium levels of a polar (hydrophilic) phase, usually water, into high levels of oil by stabilising with cationic quaternary ammonium surfactant and cosurfactant. These microemulsions have the additional advantage of remaining homogenous when diluted with spreading agents such as fatty esters. When low levels of high HLB dispersants are incorporated, the formulations bloom when added to water producing a semi-dispersing oil-in-water emulsion of fine droplet size. As bath additive concentrates therefore, the microemulsions according to the invention function as effective delivery vehicles for quaternary ammonium antibacterial agents, and for flocculating oil droplets which slowly adhere to the skin.

The microemulsion according to the invention is distinguished from known microemulsions containing similar ingredients for use in the cosmetic and pharmaceutical fields by being characterised by a sparkling, clear, homogenous system which is especially desirable for cosmetic products and also by its exceptional stability.

The microemulsion according to the invention can be used as a homogenous system for delivering relatively high levels of bath oil and water-soluble antibacterial agent. Thus the microemulsions according to the invention have particular application as bath additives and in hair care products.

EP 0 278 660 B1

The microemulsions of the present invention may comprise 30-90% of an oil, such as liquid paraffin in an amount of 50-60% by weight as the hydrophobic oily phase. The hydrophilic or polar phase is suitably water in an amount of 1-10% by weight. The cationic surfactant is generally present in an amount of 1-20% by weight.

The hydrophobic oily phase is suitably comprised of one or more hydrocarbon, mineral, animal or vegetable oils or a mixture thereof.

The hydrophobic oily phase may suitably comprise one or more liquid hydrocarbons.

The hydrophobic oily phase may also suitably comprise a triglyceride of natural extraction. Such a hydrophobic oily phase may additionally contain a synthetic triglyceride, a hydrocarbon oil or a naphthenic oil.

Among the oils comprising the hydrophobic oily phase the following are preferred:- liquid paraffin, squalane or other hydrocarbon or mineral oils, arachis, soya, castor, jojoba, olive, cottonseed, sunflower, almond, coconut or fractionated coconut or other vegetable or hydrogenated, or otherwise derivatised-vegetable oils, mink or other animal oils or any combination or admixture thereof.

The hydrophobic oily phase may comprise, for example, fractionated, hydrogenated or otherwise chemically altered triglyceride oil.

A suitable silicone oil for use as the hydrophobic oily phase is cyclomethicone.

The hydrophilic or polar phase employed in the microemulsions according to the invention may be chosen from water and monohydroxyalkanes including ethanol, propanol and isopropanol; dihydroxyalkanes including alkyl glycols wherein the alkyl group contains from 2 to 8 carbon atoms; trihydroxyalkanes including glycerol and other triols wherein the alkyl chain contains from 3 to 8 carbon atoms or any structural isomers therefrom; polyglycols such as polyethylene glycol; mannitol, sorbitol or other polyhydric liquid saccharides or any combination or admixture thereof.

The quaternary ammonium surfactant preferably has antibacterial properties. Among the quaternary ammonium surfactants the following are especially preferred:-monoalkyl trimethylammonium salts, dialkyl dimethylammonium salts, trialkyl methylammonium salts, alkyl benzyl dimethylammonium salts, benzyl alkylammonium halides, alkylpyridinium halides, alkylimidazolidine halides in which the alkyl radical contains from 6 to 24 carbon atoms; this latter alkyl radical may be in particular: a capryl, dodecyl, tetradecyl, hexadecyl, octadecyl, cetyl, stearyl, cocoyl, dodecyl-benzyl, dodecyl-phenyl, isohexadecyl, oleyl or palmitoyl radical; it may also come from tallow (hydrogenated or not), soya or copra.

The quaternary ammonium salts may be halides (chloride, bromide or iodide) sulphates, perchlorates, phosphates and other salts.

The stabilising effect of the cationic surfactant has been found to be greatest in those of aromatic character, e.g. benzalkonium chloride and benzethonium chloride form stable microemulsions from 50% aqueous solution as hereinafter indicated in Example 2, wherein the water and quaternary ammonium compound are used in a ratio of 1:1.

Cationic surfactants of aliphatic character, on the other hand, require high levels of cosurfactant to stabilise relatively low levels of water and cationic surfactant as hereinafter indicated in Example 3.

The cosurfactant or cosolubiliser used in the microemulsions according to the invention may be chosen from those of the known prior art and particularly from alcohols; ketones; aldehydes; ethers; diols; ethers of glycerol, mannitol and sorbitol; organic acids; acid salts; glyceryl ether sulphonates; alkoxamides; sulphoxides; amine salts and phosphoric acid mono- and diesters.

Especially preferred cosurfactants include oleyl alcohol, octyldodecanol, sorbitan isostearate, sorbitan monooleate and sorbitan sesquioleate.

The microemulsion according to the invention is preferably a water-in-oil emulsion which is stabilised by the cationic surfactant.

The microemulsion according to the invention contains from 0.1 to 10% by weight of a dispersant of high HLB, preferably a dispersant having a HLB value greater than 9. Suitable dispersants include Laureth 4 (polyoxyethylene (4) lauryl ether) and polyethylene glycol 400 dilaurate.

As examples of the potential use of the microemulsions according to the invention the following are especially indicated:- controlled delivery systems in topical pharmaceuticals; as water-dispersible antibacterial oily bath additives; delivery systems for essential oils and quaternary ammonium hair conditioners in cosmetics.

The microemulsions according to the invention may also include one or more active ingredients. For example, the active ingredient may be an antibacterial agent such as 8-hydroxyquinoline or triclosan.

Other excipients for use in the microemulsion according to the invention include emollients such as acetylated lanolin alcohols which improve the spreading and feel of the microemulsion when applied to the skin.

3

The invention will be further illustrated by the following Examples.

EXAMPLE 1

| Ingredient | % w/w |
|---|---|
| Arachis oil | 53.00 |
| Isopropyl palmitate | 11.60 |
| Benzalkonium chloride BP (50% aqueous solution) | 9.40 |
| Oleyl alcohol | 15.00 |
| Laureth 4 | 11.00 |

The microemulsion was prepared by heating the ingredients to 40°C, with stirring, until clear and homogenous. The microemulsion thereby produced can be used as a dispersible antibacterial bath additive.

Example 3

| Ingredient | % |
|---|---|
| Light liquid paraffin | 78.60 |
| Sorbitan monooleate | 13.70 |
| Polyethylene glycol 400 dilaurate | 6.20 |
| Lauryltrimethylammonium chloride | 0.07 |
| Water | 0.13 |

The microemulsion was prepared from the above ingredients by the procedure of Example 1. The composition thereby produced is suitable for use as a dispersible antibacterial bath additive.

Example 4

A microemulsion having antibacterial properties was formed, according to the procedure of Example 1, from the following ingredients:-

| Ingredient | % |
|---|---|
| Light liquid paraffin | 56.50 |
| Isopropyl myristate | 17.00 |
| Laureth 4 | 6.50 |
| Oleyl alcohol | 8.00 |
| Benzalkonium chloride BP | 10.00 |
| 8- Hydroxyquinoline | 2.00 |

Example 5

4

| Ingredient | % w/w |
|---|---|
| Light liquid paraffin | 68.56 |
| Cyclomethicone | 18.37 |
| Oleyl alcohol | 7.51 |
| Benzalkonium chloride | 1.59 |
| Water | 1.59 |
| Laureth 4 | 2.38 |

A microemulsion was prepared from the above ingredients by heating the ingredients, with the exception of the cyclomethicone, to 65°C until a clear medium was obtained. The cyclomethicone was added after cooling of the medium to 40°C. A semi-dispersing bath oil containing cyclomethicone was thereby obtained.

Example 6

| Ingredient | % w/w |
|---|---|
| Light liquid paraffin | 79.68 |
| Sorbitan monooleate | 12.79 |
| Polyethylene glycol 400 dilaurate | 6.42 |
| Lauryldimethylbenzylammonium chloride | 0.36 |
| Water | 0.19 |

The above ingredients were mixed with vigorous stirring until a clear microemulsion was obtained. An oily hair additive was thereby obtained.

Example 7

| Ingredient | % w/w |
|---|---|
| Light liquid paraffin | 50.74 |
| Isopropyl palmitate | 16.42 |
| Acetylated lanolin alcohols | 3.86 |
| Laureth 4 | 6.28 |
| Oleyl alcohol | 7.73 |
| Benzalkonium chloride | 5.80 |
| Water | 5.80 |
| Polyethylene glycol | 3.38 |

The above ingredients were mixed with slow stirring and heating until a clear homogenous medium was obtained. The product thereby obtained can be used as an antibacterial bath additive.

Example 8

| Ingredient | % w/w |
|---|---|
| Light liquid paraffin | 52.40 |
| Acetylated lanolin alcohols | 4.00 |
| Isopropyl palmitate | 17.00 |
| Oleyl alcohol | 6.00 |
| Laureth 4 | 6.50 |
| Triclosan | 2.00 |
| Benzalkonium chloride | 6.00 |
| Fragrance | 0.10 |
| Water | 6.00 |

A microemulsion of the above composition was prepared by gently heating a mixture of all of the above ingredients and stirring until clear.

The physical stability of the microemulsion thereby prepared was assessed by determining % light transmission at 730 nm before and after storage for up to 18 months at 4°C, room temperature (RT), 32°C and 42°C. The results obtained for three different batches of the microemulsion are given in the following Table. The stability of the microemulsion when so assessed was found to be excellent as indicated in the Table.

## TABLE

### Batch 1

| Time | 4°C | RT | 32°C | 42°C |
|---|---|---|---|---|
| Initial | − | 102.0 | − | − |
| 1 Month | 102.5 | 102.5 | 102.3 | 102.0 |
| 2 Months | 102.1 | 102.0 | 102.0 | 101.7 |
| 3 Months | 101.7 | 102.1 | 101.8 | 102.0 |
| 6 Months | 100.3 | 100.6 | 101.5 | 101.7 |
| 12 Months | 101.9 | 100.7 | 100.0 | 99.0 |
| 18 Months | 100.0 | 100.0 | 88.0 | − |

### Batch 2

| Time | 4°C | RT | 32°C | 42°C |
|---|---|---|---|---|
| Initial | − | 102.0 | − | − |
| 1 Month | 102.1 | 102.7 | 100.6 | 100.5 |
| 2 Months | 101.2 | 101.7 | 100.1 | 100.2 |
| 3 Months | 102.6 | 102.4 | 102.7 | 103.0 |
| 6 Months | 100.1 | 102.2 | 101.3 | 102.1 |
| 12 Months | 101.9 | 100.2 | 100.0 | 99.9 |
| 18 Months | 100.0 | 100.3 | 98.0 | − |

## TABLE contd.

### Batch 3

| Time | 4°C | RT | 32°C | 42°C |
|---|---|---|---|---|
| Initial | – | 100.2 | – | – |
| 1 Month | 100.0 | 100.3 | 101.3 | 99.1 |
| 2 Months | 97.7 | 100.7 | 99.9 | 100.7 |
| 3 Months | 100.2 | 100.7 | 100.6 | 101.9 |
| 6 Months | 101.5 | 101.1 | 101.4 | 101.1 |
| 12 Months | 105.0 | 102.1 | 104.5 | 102.0 |
| 18 Months | 101.4 | 101.4 | 99.3 | – |

**Claims**

**Claims for the following Contracting States : AT, CH, DE, FR, GB, GR, IT, LI, LU, NL, SE**

1. A microemulsion for topical application to the hair and skin comprising a hydrophobic oily phase, a hydrophilic phase, a cationic quaternary ammonium surfactant, a cosurfactant and a dispersant characterised in that the dispersant has a high HLB of at least 9 and in that microemulsion is clear and homogeneous and contains at least 20% by weight of said hydrophobic oily phase, 0.01-20% by weight of said hydrophilic phase, 0.01-20% of said cationic quaternary ammonium surfactant, 0.1 to 10% by weight of said dispersant and sufficient of said cosurfactant to generate and secure homogeneity and clarity of the microemulsion.

2. A microemulsion according to Claim 1, characterised in that the hydrophobic oily phase is comprised of one or more hydrocarbon, mineral, animal or vegetable oils or a mixture thereof.

3. A microemulsion according to Claim 1 or 2, characterised in that the hydrophobic oily phase comprises one or more liquid hydrocarbons or fractionated, hydrogenated or otherwise chemically altered triglyceride oil.

4. A microemulsion according to Claim 1 or 2, characterised in that the hydrophobic oily phase comprises a triglyceride of natural extraction.

5. A microemulsion according to Claim 5, characterised in that the hydrophobic oily phase additionally contains a synthetic triglyceride, a hydrocarbon oil or a naphthenic oil.

6. A microemulsion according to any preceding claim, characterised in that the hydrophilic phase is selected from: water; an alcohol in which the alkyl chain contains from 1 to 3 carbon atoms; a glycol wherein the major alkyl chain contains from 2 to 8 carbon atoms; glycerol or other trihydroxyalkane wherein the alkyl chain contains from 3 to 8 carbon atoms; a polyglycol; or a polyhydric liquid saccharide or a mixture thereof.

7. A microemulsion according to any preceding claim, characterised in that the quaternary ammonium surfactant is a quaternary ammonium salt containing at least one alkyl group having from 6 to 24 carbon atoms, said salt being selected from a halide, a sulphate, a perchlorate or a phosphate.

8

EP 0 278 660 B1

**8.** A microemulsion according to any preceding claim, characterised in that the cosurfactant is selected from: alcohols; ketones; aldehydes; ethers; diols; ethers of glycerol, mannitol and sorbitol; organic acids; acid salts; glyceryl ether sulphonates; alkoxamides; sulphoxides; amine salts and phosphoric acid mono- and diesters.

**9.** A microemulsion according to Claim 8, characterised in that the cosurfactant is selected from oleyl alcohol, octyldodecanol, sorbitan isostearate, sorbitan monooleate and sorbitan sesquioleate.

**10.** A microemulsion according to any preceding claim characterised in that it is in the form of a water-in-oil emulsion.

**11.** Use of a microemulsion according to any preceding claim in the preparation of a pharmaceutical preparation for topical application to the hair or skin.

**Claims for the following Contracting State : ES**

**1.** A process for the preparation of a microemulsion for topical application to the hair and skin comprising a hydrophobic oily phase, a hydrophilic phase, a cationic quaternary ammonium surfactant, a cosurfactant and a dispersant characterised in that the dispersant has a high HLB of at least 9, and in that the microemulsion is clear and homogeneous and contains at least 20% by weight of said hydrophobic oily phase, 0.01-20% by weight of said hydrophilic phase, 0.01-20% of said cationic quaternary ammonium surfactant, 0.1-10% by weight of said dispersant and sufficient of said cosurfactant to generate and secure homogeneity and clarity of the microemulsion, the process comprising admixing the ingredients.

**2.** A process according to Claim 1, characterised in that the hydrophobic oily phase is comprised of one or more hydrocarbon, mineral, animal or vegetable oils or a mixture thereof.

**3.** A process according to Claim 1 or 2, characterised in that the hydrophobic oily phase comprises one or more liquid hydrocarbons or fractionated, hydrogenated or otherwise chemically altered triglyceride oil.

**4.** A process according to Claim 1 or 2, characterised in that the hydrophobic oily phase comprises a triglyceride of natural extraction.

**5.** A process according to Claim 5, characterised in that the hydrophobic oily phase additionally contains a synthetic triglyceride, a hydrocarbon oil or a naphthenic oil.

**6.** A process according to any preceding claim, characterised in that the hydrophilic phase is selected from: water; an alcohol in which the alkyl chain contains from 1 to 3 carbon atoms; a glycol wherein the major alkyl chain contains from 2 to 8 carbon atoms; glycerol or other trihydroxyalkane wherein the alkyl chain contains from 3 to 8 carbon atoms; a polyglycol; or a polyhydric liquid saccharide or a mixture thereof.

**7.** A process according to any preceding claim, characterised in that the quaternary ammonium surfactant is a quaternary ammonium salt containing at least one alkyl group having from 6 to 24 carbon atoms, said salt being selected from a halide, a sulphate, a perchlorate or a phosphate.

**8.** A process according to any preceding claim, characterised in that the cosurfactant is selected from: alcohols; ketones; aldehydes; ethers; diols; ethers of glycerol, mannitol and sorbitol; organic acids; acid salts; glyceryl ether sulphonates; alkoxamides; sulphoxides; amine salts and phosphoric acid mono- and diesters.

**9.** A process according to Claim 8, characterised in that the cosurfactant is selected from oleyl alcohol, octyldodecanol, sorbitan isostearate, sorbitan monooleate and sorbitan sesquioleate.

**10.** A process according to any preceding claim characterised in that it is in the form of a water-in-oil emulsion.

**11.** Use of a microemulsion prepared by a process according to any preceding claim in the preparation of

9

a pharmaceutical preparation for topical application to the hair or skin.

**Patentansprüche**

**Patentansprüche für folgende Vertragsstaaten : AT, CH, DE, FR, GB, GR, IT, LI, LU, NL, SE**

1. Mikroemulsion zur örtlichen Anwendung auf das Haar und die Haut, die eine hydrophobe, ölhaltige Phase, eine hydrophile Phase, einen kationischen, quartären, Ammoniaksurfaktanten, einen Co-Surfaktanten und ein Dispersionsmittel umfaßt, dadurch gekennzeichnet, daß das Dispersionsmittel einen hohen HLB-Wert von mindestens 9 enthält, und daß die Mikroemulsion klar und homogen ist und mindestens 20 Gew.% besagter hydrophober ölhaltiger Phase, 0,00-20 Gew.% besagter hydrophiler Phase, 0,01-20% von besagtem kationischen, quartären Ammoniaksurfaktanten, 0,1 bis 10 Gew.% von besagtem Dispersionsmittel und eine ausreichende Menge von besagtem Co-Surfaktanten zur Erzeugung und Sicherung der Homogenität und Klarheit der Mikroemulsion enthält.

2. Mikroemulsion nach Anspruch 1, dadurch gekennzeichnet, daß die hydrophobe, ölhaltige Phase einen oder mehrere Kohlenwasserstoffe, mineralische, tierische oder pflanzliche Öle oder eine Mischung derselben enthält.

3. Mikroemulsion nach den Ansprüchen 1 oder 2, dadurch gekennzeichnet, daß die hydrophobe, ölhaltige Phase einen oder mehrere flüssige Kohlenwasserstoffe oder fraktioniertes, hydriertes oder auf andere Weise chemisch verändertes Triglyzeridöl enthält.

4. Mikroemulsion nach den Ansprüchen 1 oder 2, dadurch gekennzeichnet, daß die hydrophobe, ölhaltige Phase ein aus natürlichen Extrakten bestehendes Triglyzerid enthält.

5. Mikroemulsion nach Anspruch 5 (richtigerweise 4), dadurch gekennzeichnet, daß die hydrophobe, ölhaltige Phase zusätzlich ein synthetisches Triglyzerid, ein Kohlenwasserstofföl oder ein Naphthen enthaltendes Öl enthält.

6. Mikroemulsion nach einem der vorstehenden Ansprüche, dadurch gekennzeichnet, daß die hydrophile Phase aus folgendem selektiert wird: aus Wasser; aus einem Alkohol, bei dem die Alkylkette von 1 bis 3 Kohlenstoffatome enthält; aus einem Glykol, worin die Hauptaklylkette von 2 bis 8 Kohlenstoffatome enthält; aus Glycerin oder anderem Trihydroxylalkan, worin die Alkylkette von 3 bis 8 Kohlenstoffatome enthält; aus einem Polyglykol; oder aus einem mehrere Hydroxylgruppen enthaltenden flüssigem Saccharid, oder aus einer Mischung desselben.

7. Mikroemulsion nach einem der vorstehenden Ansprüche, dadurch gekennzeichnet, daß der quartäre Ammoniaksurfaktant ein quartäres Ammoniaksalz ist, das mindestens eine Alkylgruppe mit 6 bis 24 Kohlenstoffatomen enthält, wobei besagtes Salz aus einem Halogenid, einem Sulfat, einem Perchlorat oder einem Phosphat selektiert wird.

8. Mikroemulsion nach einem der vorstehenden Ansprüche, dadurch gekennzeichnet, daß der Co-Surfaktant aus folgendem selektiert wird: Alkoholen; Ketonen; Aldehyden; Äthern; Diolen; Äthern aus Glycerin, Mannit und Sorbit; organische Säuren; Säuresalzen; Glycerinäthersulfonaten; Alkoxamiden; Sulfoxyden; Aminsalzen und Phosphorsäuremono- und -diestern.

9. Mikroemulsion nach Anspruch 8, dadurch gekennzeichnet, daß der Co-Surfaktant aus Oleylalkohol, Oktyldodekanol, sorbithaltigem Isostearat, sorbithaltigem Monooleat und sorbithaltigem Sesquioleat selektiert wird.

10. Mikroemulsion nach einem der vorstehenden Ansprüche, dadurch gekennzeichnet, daß sie aus einer Wasser-in-Öl-Emulsion besteht.

11. Gebrauch einer Mikroemulsion nach einer der vorstehenden Ansprüche durch die Bereitung eines pharmazeutischen Präparats für die örtliche Anwendung auf das Haar oder die Haut.

**Patentansprüche für folgenden Vertragsstaat : ES**

EP 0 278 660 B1

1. Verfahren zur Bereitung einer Mikroemulsion zur örtlichen Anwendung auf das Haar und die Haut, die eine hydrophobe, ölhaltige Phase, eine hydrophile Phase, einen kationischen, quartären Ammoniaksurfaktanten, einen Co-Surfaktanten und ein Dispersionsmittel umfaßt, dadurch gekennzeichnet, daß das Dispersionsmittel einen hohen HLB-Wert von mindestens 9 enthält, und daß die Mikroemulsion klar und homogen ist und mindestens 20 Gew.% besagter hydrophober, ölhaltiger Phase, 0,01-20 Gew-% besagter hydrophiler Phase, 0,01-20% von besagtem kationischen, quartären Ammoniaksurfaktanten, 0,01-10 Gew.% von besagtem Dispersionsmittel und eine ausreichende Menge von besagtem Co-Surfaktanten zur Erzeugung und Sicherung der Homogenität und Klarheit der Mikroemulsion enthält, wobei das Verfahren die Beimischung der Zutaten umfaßt.

2. Verfahren nach Anspruch 1, dadurch gekennzeichnet, daß die hydrophobe, ölhaltige Phase einen oder mehrere Kohlenwasserstoffe, mineralische, tierische oder pflanzliche Öle oder eine Mischung derselben enthält.

3. Verfahren nach den Ansprüchen 1 oder 2, dadurch gekennzeichnet, daß die hydrophobe, ölhaltige Phase einen oder mehrere flüssige Kohlenwasserstoffe oder fraktoniertes, hydriertes oder auf andere Weise chemisch verändertes Triglyzeridöl enthält.

4. Verfahren nach den Ansprüchen 1 oder 2, dadurch gekennzeichnet, daß die hydrophobe, ölhaltige Phase ein aus natürlichen Extrakten bestehendes Triglyzerid enthält.

5. Verfahren nach Anspruch 5 (richtigerweise 4), dadurch gekennzeichnet, daß die hydrophobe, ölhaltige Phase zusätzlich ein synthetisches Triglyzerid, ein Kohlenwasserstofföl oder ein Naphthen enthaltendes Öl enthält.

6. Verfahren nach einem der vorstehenden Ansprüche, dadurch gekennzeichnet, daß die hydrophile Phase aus folgendem selektiert wird: aus Wasser; aus einem Alkohol, bei dem die Alkylkette von 1 bis 3 Kohlenstoffatome enthält; aus einem Glykol, worin die Hauptalkylkette von 2 bis 8 Kohlenstoffatome enthält; aus Glycerin oder anderem Trihydroxylalkan, worin die Alkylkette von 3 bis 8 Kohlenstoffatome enthält; aus einem Polyglykol; oder aus einem mehrere Hydroxylgruppen enthaltenden flüssigen Saccharid, oder aus einer Mischung desselben.

7. Verfahren nach einem der vorstehenden Ansprüche, dadurch gekennzeichnet, daß der quartäre Ammoniaksurfaktant ein quartäres Ammoniaksalz ist, das mindestens eine Alkylgruppe mit 6 bis 24 Kohlenstoffatomen enthält, wobei besagtes Salz aus einem Halogenid, einem Sulfat, einem Perchlorat oder einem Phosphat selektiert wird.

8. Verfahren nach einem der vorstehenden Ansprüche, dadurch gekennzeichnet, daß der Co-Surfaktant aus folgendem selektiert wird: Alkoholen; Ketonen;Aldehyden; Äthern; Diolen; Äthern aus Glycerin, Mannit und Sorbit; organischen Säuren; Säuresalzen; Glycerinäthersulfonaten; Alkoxamiden; Sulfoniden; Aminsalzen und Phosphorsäuremono- und -diestern.

9. Verfahren nach Anspruch 8, dadurch gekennzeichnet, daß der Co-Surfaktant aus Oleylalkohol, Oktyldodekanol, sorbithaltigem Isostearat, sorbithaltigem Monooleat und sorbithaltigem Sesquioleat selektiert wird.

10. Verfahren nach einem der vorstehenden Ansprüche, dadurch gekennzeichnet, daß es aus Wasser-in-Öl-Emulsion besteht.

11. Gebrauch einer Mikroemulsion, bereitet nach einem in einer der vorstehenden Ansprüche beanspruchten Verfahren durch die Bereitung eines pharmazeutischen Präparats für die örtliche Anwendung auf das Haar oder die Haut.

**Revendications**

**Revendications pour les Etats contractants suivants : AT, CH, DE, FR, GB, GR, IT, LI, LU, NL, SE**

1. Microémulsion pour application locale sur les cheveux et la peau comprenant une phase huileuse hydrophobe, une phase hydrophile, un surfactant ammonium quaternaire cationique, un cosurfactant et

11

un agent de dispersion, caractérisée par le fait que l'agent de dispersion a un HLB élevé d'au moins 9 et la microémulsion est claire et homogène et contient au moins 20 % en poids de ladite phase huileuse hydrophobe, 0,01 à 20 % en poids de ladite phase hydrophile, 0,1 à 20 % en poids dudit surfactant ammonium quaternaire cationique, 0,1 à 10 % en poids dudit agent de dispersion et suffisamment dudit cosurfactant pour créer et assurer l'homogénéité et la clarté de la microémulsion.

2. Microémulsion selon la revendication 1, caractérisée par le fait que la phase huileuse hydrophobe est constituée d'un ou plusieurs hydrocarbures ou huiles minérales, animales ou végétales ou d'un mélange de ceux-ci

3. Microémulsion selon les revendications 1 ou 2, caractérisée par le fait que la phase huileuse hydrophobe comprend un ou plusieurs hydrocarbures liquides ou une huile de triglycéride fractionné, hydrogéné, ou modifié chimiquement d'une autre façon.

4. Microémulsion selon les revendications 1 ou 2, caractérisée par le fait que la phase huileuse hydrophobe comprend un triglycéride extrait naturellement.

5. Microémulsion selon la revendication 5, caractérisée par le fait que la phase liquide hydrophobe contient en plus un triglycéride synthétique, un hydrocarbure ou une huile naphténique.

6. Microémulsion selon l'une quelconque des revendications précédentes, caractérisée par le fait que la phase hydrophile est choisie parmi : l'eau, un alcool dont la chaîne alkyle contient 1 à 3 atomes de carbone, un glycol dont la chaîne alkyle principale contient 2 à 8 atomes de carbone ; du glycérol ou un autre trihydroxyalcane dont la chaîne alkyle contient de 3 à 8 atomes de carbone ; un polyglycol ou un saccharide liquide polyhydrique ou un mélange de ceux-ci.

7. Microémulsion selon l'une quelconque des revendications précédentes, caractérisée par le fait que le surfactant ammonium quaternaire est un sel d'ammonium quaternaire contenant au moins un groupe alkyle de 6 à 24 atomes de carbone, ledit sel étant choisi parmi un halogénure, un sulfate, un perchlorate ou un phosphate.

8. Microémulsion selon l'une quelconque des revendications précédentes, caractérisée par le fait que le cosurfactant est choisi parmi : des alcools ; des cétones ; des aldéhydes ; des éthers ; des diols ; des éthers de glycérol, de mannitol et de sorbitol ; des acides organiques ; des sels acides ; des sulfonates d'éther glycérylique ; des alkoxamides ; des sulfoxides ; des mono- et diesters de sels d'amines et d'acide phosphorique.

9. Microémulsion selon la revendication 8, caractérisée par le fait que le cosurfactant est choisi parmi l'alcool oléylique, l'octyldodécanol, l'isostéarate de sorbitane, le momooléate de sorbitane et le sesquioléate de sorbitane.

10. Microémulsion selon l'une quelconque des revendications précédentes, caractérisée par le fait, qu'elle se trouve sous forme d'une émulsion eau-dans-l'huile.

11. Utilisation d'une microémulsion selon l'une quelconque des revendications précédentes dans la préparation d'une composition pharmaceutique destinée à l'application locale sur les cheveux ou la peau.

**Revendications pour l'Etat contractant suivant : ES**

1. Procédé pour la préparation d'une microémulsion pour application locale sur les cheveux et la peau comprenant une phase huileuse hydrophobe, une phase hydrophile, un surfactant ammonium quaternaire cationique, un cosurfactant et un agent de dispersion, caractérisé par le fait que l'agent de dispersion a un HLB élevé d'au moins 9 et la microémulsion est claire et homogène et contient au moins 20 % en poids de ladite phase huileuse hydrophobe, 0,01 à 20 % en poids de ladite phase hydrophile, 0,1 à 20 % en poids dudit surfactant ammonium quaternaire cationique, 0,1 à 10 % en poids dudit agent de dispersion et suffisamment dudit cosurfactant pour créer et assurer l'homogénéité et la clarté de la microémulsion.

2.  Procédé selon la revendication 1, caractérisé par le fait que la phase huileuse hydrophobe est constituée d'un ou plusieurs hydrocarbures ou huiles minérales, animales ou végétales, ou d'un mélange de ceux-ci.

3.  Procédé selon les revendications 1 ou 2, caractérisé par le fait que la phase huileuse hydrophobe comprend un ou plusieurs hydrocarbures liquides ou une huile de triglycéride fractionné hydrogéné, ou modifié chimiquement d'une autre façon.

4.  Procédé selon les revendications 1 ou 2, caractérisé par le fait que la phase huileuse hydrophobe comprend un triglycéride extrait naturellement.

5.  Procédé selon la revendication 5, caractérisé par le fait que la phase huileuse hydrophobe contient en plus un triglycéride synthétique, un hydrocarbure ou une huile naphténique.

6.  Procédé selon l'une quelconque des revendications précédentes, caractérisé par le fait que la phase hydrophile est choisie parmi : l'eau, un alcool dont la chaîne alkyle contient 1 à 3 atomes de carbone ; un glycol dont la chaîne alkyle principale contient 2 à 8 atomes de carbone ; du glycérol ou un autre trihydroxyalcane dont la chaîne alkyle contient de 3 à 8 atomes de carbone ; un polyglycol ou un saccharide liquide polyhydrique ou un mélange de ceux-ci.

7.  Procédé selon l'une quelconque des revendications précédentes, caractérisé par le fait que le surfactant ammonium quaternaire est un sel d'ammonium quaternaire contenant au moins un groupe alkyle de 6 à 24 atomes de carbone, ledit sel étant choisi parmi un halogénure, un sulfate, un perchlorate ou un phosphate.

8.  Procédé selon l'une quelconque des revendications précédentes, caractérisé par le fait que le cosurfactant est choisi parmi : des alcools ; des cétones ; des aldéhydes ; des éthers ; des diols ; des éthers de glycérol, de mannitol et de sorbitol ; des acides organiques ; des sels acides ; des sulfonates d'éther glycérylique ; des alkoxamides ; des sulfoxides ; des mono- et diesters de sels d'amines et d'acide phosphorique.

9.  Procédé selon la revendication 8, caractérisé par le fait que le cosurfactant est choisi parmi l'alcool oléylique, l'octyldodécanol, l'isostéarate de sorbitane, le momooléate de sorbitane et le sesquioléate de sorbitane.

10. Procédé selon l'une quelconque des revendications précédentes, caractérisé par le fait, qu'elle se trouve sous forme d'une émulsion eau-dans-l'huile.

11. Utilisation d'une microémulsion préparée par un procédé selon l'une quelconque des revendications précédentes dans la préparation d'une composition pharmaceutique destinée à l'application locale sur les cheveux ou la peau.